# EUROPEAN PATENT SPECIFICATION

(11) **EP 0 608 196 B1**
(45) Date of publication and mention of the grant of the patent: **12.09.2001**
(21) Application number: 94610007.0
(22) Date of filing: 21.01.1994
(51) Int. Cl.: G01N 33/02, G01N 3/56, A23N 17/00

(54) **Method and measuring apparatus for the testing of especially fodder pellets**
Verfahren und Messvorrichtung zum Testen insbesondere von Futtertabletten
Méthode et appareil de mesure pour tester notamment des pellets de fourage

(30) Priority: 22.01.1993 DK 7793; 10.03.1993 DK 27493
(43) Date of publication of application: 27.07.1994
(73) Proprietor: NORVIDAN OVERSEAS A/S, 5230 Odense M (DK)
(72) Inventor: Larsen, Ebbe Busch, DK-5220 Odense SÖ (DK); Hjernö, John, DK-5210 Odense NV (DK)
(74) Representative: Larsen, Hans Ole

(56) References cited:
- EP-A- 0 085 507
- GB-A- 2 181 559

## Description

The present invention relates to a method for the testing of feed pellets which are extruded in a production unit, where the pellets are removed, cooled and dried before the testing of their consistency through the following steps:
a) A sample is weighed and the result is registered as a 1st weighing,
b) the sample is swirled around and blown through to remove dust and loosely-sitting particles from the sample,
c) the sample is weighed and the result is registered as 2nd weighing,
d) 1st and 2nd weighings are compared as a measure of the consistency of the sample,
e) the sample is replaced by a subsequent sample.

The invention also relates to a tester for use in the execution of the disclosed method, said tester being of the kind which has first pneumatic elements for cooling and drying, and other pneumatic elements for the blowing through and the swirling of a sample of feed pellets, weighing elements for weighing the relevant sample, storage elements for storing the results of the weighings and calculating elements for the comparison of the results of the weighings which are obtained before and after the blowing through and swirling of a sample.

A method and tester of the kind disclosed are known from EP patent specification no. 0 040 406 and English patent specification no. GB-A-2 181 559.

With the known technique, samples of feed pellets are removed at short intervals from the current production of feed pellets and are examined for their consistency, in that after a first weighing of a sample, which consists of a number of feed pellets, the sample is exposed to a mechanical process for the removal of dust and loose particles, after which the sample is re-weighed and the difference between the two weighings is displayed as a measure of the consistency of the sample.

When the testing of a sample is concluded, the relevant sample is replaced by a subsequent sample, after which the testing sequence is continued.

A pellet durability tester comprising an air recirculation path into which pellets may be introduced to determine their durability, and means for establishing the relative quantities of whole pellets in the sample before and after testing and thereby the durability of the pellets, is known from European patent appln. EP 0085507.

The tester disclosed therein comprises means for at least partly drying and cooling pellets held in the holding means. Provided that the conditions under which the batch is dried and cooled in the holding means are consistent from sample to sample, the need to cool and dry the pellets prior to their introduction into the tester is eliminated.

Feed pellets are produced by pressing the material through holes while the material is heated by the introduction of steam at high temperature, and the samples must therefore be cooled and dried before the testing. This cooling and drying takes a long time because of the high temperature and high degree of moisture.

The results of the tests on the individual samples are used to regulate the production of the feed pellets in order to achieve an optimal consistency, but since the individual samples must be cooled and dried before the testing, these results are available only after inconveniently-long intervals of time.

If, during the testing sequence of a sample, it is ascertained that the current production is of unsatisfactory consistency, for reasons of the large production capacity in the production plant there will be produced a large amount of feed pellets which, as a consequence of the unsatisfactory consistency, must be returned for re-processing in the production plant.

This disadvantage may well be obviated by the use of several testers, which are used in parallel with one another, but this is inexpedient when the testers are coupled together with the production plant, the reason being that a tester's pulses for the control of the production plant can have a disturbing effect on the pulses from the remaining testers.

It is the object of the present invention to present a method and a tester for the use in the execution of the method of such a kind that the disclosed disadvantages of the known technique can be overcome, so that with a single tester it is possible to achieve a continuous testing and the control of a production plant of the mentioned kind without any unnecessarily long reaction time between the ascertainment of an unsatisfactory consistency and the improvement of this consistency.

This object is achieved by a method of the kind disclosed in the preamble, said method according to the invention being characterized in that the subsequent sample is cooled and dried concurrently with the described steps a) - e).

According to the invention, the tester for use in the execution of the method is characterized by the first pneumatic elements comprising a tube piece which in a position of use surrounds an upper container provided with a perforated wall, which via a closable opening is connected to a lower container provided with closed wall and a downwardly-facing opening, and in the pipe piece between the upper and the lower container a channel for the supply of cooling/drying air to and through the perforated wall of the upper container and through a sample of feed pellets deposited in this container, and in that the weighing elements consist of a weighing funnel with a perforated wall which at the bottom has a sieve equipped with a ring, said weighing funnel at its upper edge being pivotably suspended around a pivot in a forked weighing arm, the other end of which is connected via an axle to a weighing cell and is pivotable between a first position, where the weighing funnel abuts against and connects to the circumference of said opening of the lower container, and a second position where the weighing funnel is at a distance from said opening and can turn freely around the pivots, in which said second position the weighing cell can be activated for the weighing of the sample, that the other pneumatic elements comprise an air nozzle with an air pipe which can be removed from the weighing funnel, in that said air nozzle and pipe in a first position and in the weighing arm's first position lie up against the ring provided with a sieve and being arranged to lead a stream of air through the sample deposited in the weighing funnel and in the lower container, and which in a second position and in the weighing arm's second position is at a distance from the ring and permits the weighing funnel to be turned, and in that the tester has elements for turning the weighing funnel more than 90° away from a position of the weighing arm determined by gravity for the pouring out of a sample deposited therein.

The dependent claims disclose advantageous embodiments of the tester according to the invention.

The method and the tester according to the invention will be explained more fully in the following detailed description with reference to the drawing showing a preferred embodiment of the tester according to the invention, where
- fig. 1: shows a perspective drawing of a preferred embodiment of the tester according to the invention, in that certain internal parts of the tester are shown, and
- fig. 2 - 8: show schematic views of seven consecutive steps during the function of the tester shown in fig. 1 in the execution of the method according to the invention, and
- fig. 9: shows the immediate continuation of the cycle shown in the figures 2-8.

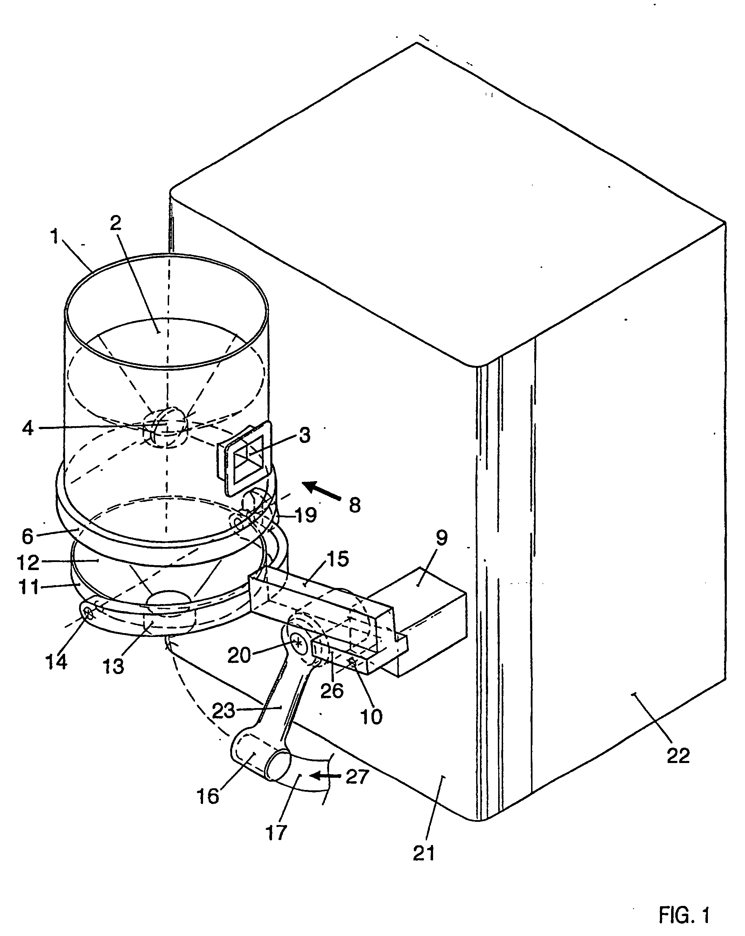

Reference is first made to figure 1.

A tester according to the invention, for the testing of feed pellets, comprises a box-shaped housing 22 having a front 21 which supports the tester's accessible elements which serve to receive samples of feed pellets 7a, 7b, 7c, --, which are to be tested. The individual samples comprising several feed pellets can weigh, for example, around 200 grams.

In an advantageous embodiment, the housing 22 with the front 21 supporting the accessible elements can have main dimensions of, for example, 525 x 500 x 370 mm, and the tester is intended to be positioned in connection with a production plant where the feed pellets are produced, in that this positioning can either be in a laboratory at the place of production or in any other expedient place. The production plant can be of the kind which, for example, is known from DK patent specification no. 140 303.

The said accessible elements are a cylindrical tube piece 1 having an axis which is substantially vertical in the tester's position of use. The tube piece 1 has a funnel arrangement comprising an upper conical funnel 2 which tapers downwards and ends in an aperture in the form of a ring 4a with a circular opening which is provided with a pivotably mounted rotary valve 4. The walls of the funnel 2 are perforated with holes which must be less in diameter than the smallest of the pellets to be tested. Therefore, the funnel 2 can with advantage be replaceable according to requirements and exist in a number of versions having holes with diameters of between, for example, 2 to 8 mm.

The rotary valve 4 is a flat cylindrical disk which is arranged to be able to be turned around an axis through the plane of the ring 4a, between a closed position in which the opening in the ring 4a is blocked by the rotary valve, and an open position in which the plane of the disk extends parallel with the axis of the funnel 2 or the pipe piece 1, so that any possible contents of the funnel 2 can flow down and out through the opening in the ring.

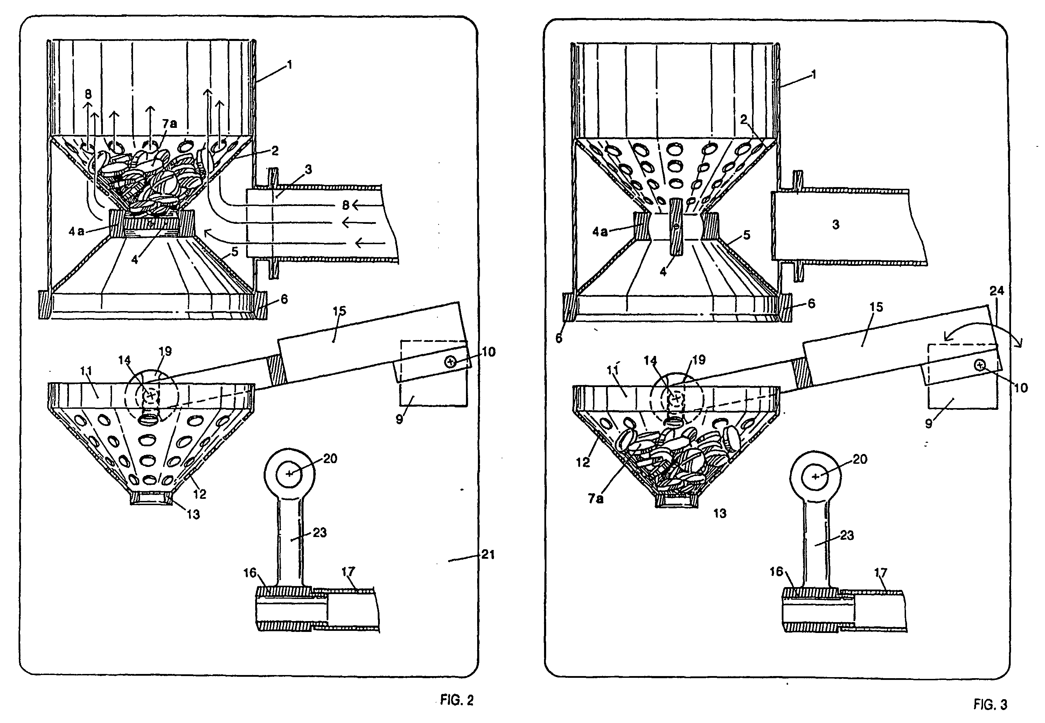

The funnel arrangement also comprises a hollow truncated cone 5 (see figs. 2-8) with its smallest diameter facing upwards and connected to the mouth of the funnel 2 by means of said ring 4a which contains the rotary valve 4. The walls of the truncated cone are unbroken.

The largest diameter on both the funnel 2 and the truncated cone 5 corresponds to the inside diameter of the pipe piece 1, and the funnel and the truncated cone abut tightly. against the inner wall of the pipe piece 1.

To the pipe piece 1, opposite the ring 4a, there is connected the one end of a channel 3 which at its other end is connected to a not-shown blower of a commonly-known design, whereby air can be blown through the channel 3 into the pipe piece 1 and through the perforated wall of the funnel 2. For the sake of clarity, the channel 3 is shown in the figures only by its last portion at the pipe piece 1, and as if this last portion extends parallel with the front 21 of the housing 22. Instead, however, it will be understood that it is expedient for the channel 3 to face directly in towards the front 21 and be connected to a blower disposed in the housing 22.

At the bottom, the pipe piece 1 terminates in an end-ring 6 which can form the contact surface for a weighing funnel 11, 12, 13 as described in the following.

The weighing funnel 11, 12, 13 is conical and enclosed at the top by a first ring 11, and at the bottom by a second ring 13 having a diameter which is less than the diameter of the first ring 11. The wall 12 of the funnel between the rings 11, 13 is perforated in the same manner as the wall of the upper funnel 2, and also exists in several selectable sizes in conformity with the upper funnel 2. The ring 13 is provided with a net which constitutes a sieve with a hole size which corresponds to the perforations in the funnel 2 and in the wall 12.

The accessible elements on the front 21 of the housing 22 also comprise a weighing arm 15 which is pivotably suspended around an axle 10 which extends at right-angles out through the front 21 and into the housing 22 where it is connected to a weighing cell 9. The weighing 15 arm can thus execute a limited turning movement between a first and a second position in a plane parallel with the front 21 of the housing 22.

The free end of the weighing arm 15 is forked in order to be able to grip around the first ring 11 which forms the top of the weighing funnel 12. On diametrically-opposite sides of the first ring 11 there are two pins 14, said two pins 14 constituting an axle around which the weighing funnel 11, 12, 13 can turn freely in the forked end of the weighing arm 15, so that the weighing funnel, under the influence of gravity, can assume a position in which its axis is vertical.

In the first position of the weighing arm 15, the weighing funnel's first ring 11 lies up against the end-ring 6 which terminates the bottom of the pipe piece 1, and in the second position the weighing funnel 11, 12, 13 is disposed at such a distance from the end-ring 6 that the weighing funnel 11, 12, 13 can turn a little more than 90° in relation to the weighing arm 15.

This turning movement of the weighing funnel 11, 12, 13 around the pins 14 is achieved by configuring the end of the pin 14 which faces the front 21 of the housing 22 as a square which, in said second position of the weighing arm 15, can engage with a turning notch 19 in the end of a shaft which extends out through the front 21. This shaft is arranged to be able to turn the weighing funnel 11, 12, 13 the said angle of a little more than 90°, and also in this turned position to be able to turn quickly forwards and backwards within a small angle around its axis in order to produce rapid oscillations or vibrations of the weighing funnel 11, 12, 13 so that any possible contents can be shaken out.

The downwardly-facing end of the second ring 13 in the weighing funnel is configured for abutment against an air nozzle 16 which can be turned between an upper and a lower position in a plane parallel with the front 21 of the housing 22, this being effected by means of an arm 23 on the end of a shaft 20 protruding from the housing 22. In its upper position, the air nozzle 16 lies against the second ring 13, and when the air nozzle is in its lower position the arm 23 is turned so far down that the air nozzle 16 does not obstruct the weighing funnel 11, 12, 13 in the second position of the weighing arm 15, not even when the weighing funnel has to be turned more than 90° in relation to the weighing arm. The shaft 20 can turn the arm 23 between the upper and the lower positions by means of the motor 26 placed in the housing 22 as shown in figure 1 of the drawing.

The end of the air nozzle 16 facing away from the second ring 13 is connected to a pipe 17, which in turn is in connection either with the blower in the housing 22 or a special blower, so that air can be blown through the pipe 17 and the air nozzle 16 through the second ring 13 up into the weighing funnel 11, 12, 13 and its possible contents when the weighing funnel is in abutment with its first ring 11 against the end-ring 6 in the funnel arrangement 1.

Reference is now made to figs. 2 to 8, which show the individual steps in the method according to the invention.

In figure 2 the tester according to the invention is shown in a start position upon start-up of a control of a production of feed pellets. In the start position, the rotary valve 4 is turned to block the passage through the ring 4a in the upper conical funnel 2, and the weighing arm 15 is lowered to its said second position where the weighing funnel 11, 12, 13 is at a distance from the end-ring 6 in the lower part of the pipe piece 1.

In a first step (see fig. 2), a sample 7a of feed pellets to be tested is placed in the upper conical funnel 2, from which the sample 7a is prevented from falling down through the ring 4a by the closed rotary valve 4.

The blower in the housing 22 is started so that a stream of cooling/drying air is led at steplessly-variable intervals of time through the channel 3 and into the intermediate space defined by the inner wall of the pipe piece 1, the funnel 2 and the hollow truncated cone 5. The blown-in air can only escape through the perforations in the funnel 2 and further to the surroundings by permeating the sample 7a, whereby during the course of a suitable period of time the sample is cooled/dried to a satisfactory degree. The path taken by the stream of air is shown by the arrows 8.

This variable period can be between 0 and 5 minutes, and the cooling/drying time is typically 2-3 minutes.

In a second step (see fig. 3), which is performed after the completion of the cooling/drying, the rotary valve 4 is opened and the sample 7a falls down into the weighing funnel 11, 12, 13. The weighing cell 9 is activated, whereby the sample 7a is weighed for the first time and the result is registered. This first weighing is indicated by the double arrow 24.

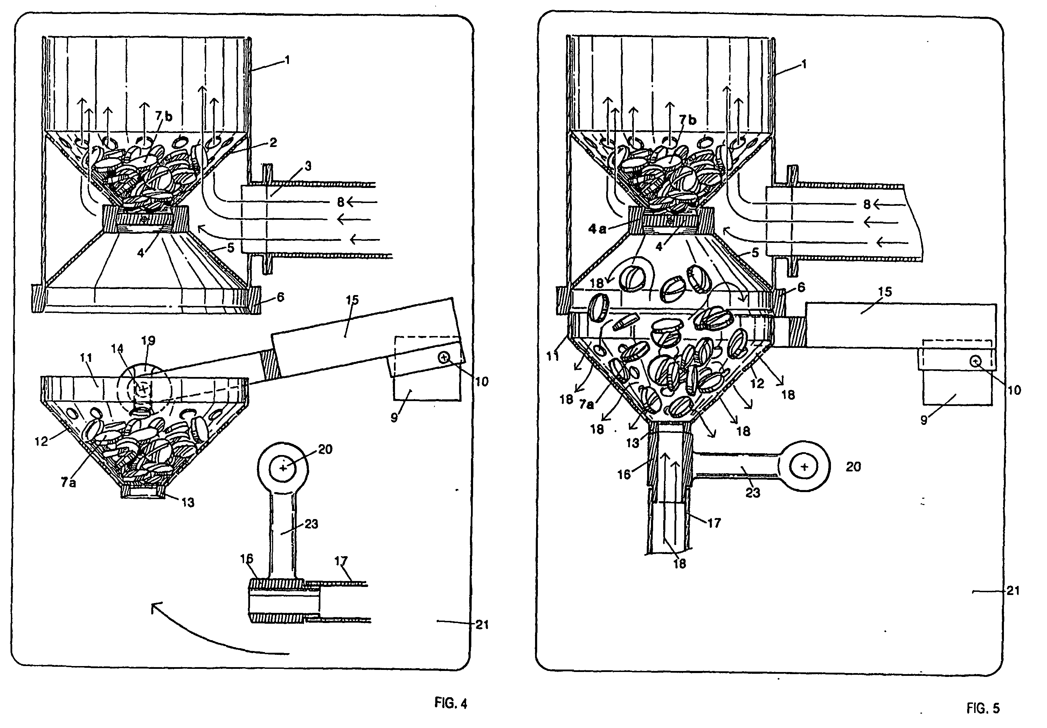

In a third step (see fig. 4), a new sample 7b is placed in the upper funnel 2 and the blower is re-started to provide a stream of cooling/drying air for the cooling/drying of the new sample 7b, as shown by the arrows 8.

In a fourth step (see fig. 5), the weighing funnel 11, 12, 13 is raised by movement of the weighing arm 15 back to its first position, so that the first ring 11 again lies against the end-ring 6 at the bottom of the funnel arrangement 1. The air nozzle 16 and the connected pipe 17 are turned around the axle 20 into abutment with the second ring 13 of the weighing funnel, and the blower in the housing 22 is opened so that a strong stream of air flows up through the weighing funnel 11, 12, 13, whereby the first sample 7a is lifted and swirled around in the space defined by the weighing funnel and the truncated cone 5, hereby cleaning the sample 7a of dust. This stream of air can escape from said space through the perforations in the wall 12 of the weighing funnel, as shown by the arrows 8. Concurrently herewith, a stream of cooling/drying air continues to be blown through the second sample 7b in the upper conical funnel 2, as shown by the arrows 8.

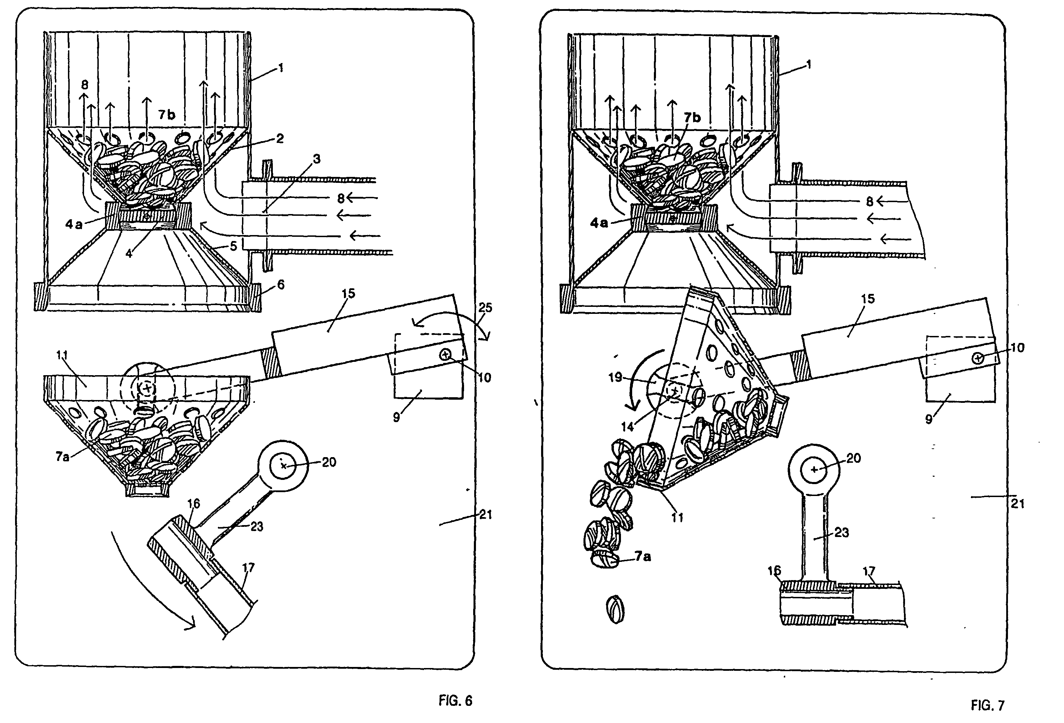

In a fifth step (see fig. 6), the air nozzle 16 and the pipe 17 are removed from the weighing funnel, and the weighing arm 15 is lowered to its second position, so that the weighing funnel 11, 12, 13 is brought into the weighing position. The weighing cell 9 is activated again for the second weighing of the first sample 7a, and the results of the first and the second weighings are compared as a link in the testing of the feed pellets. This second weighing is indicated by the double arrow 25.

In a sixth step (see fig. 7), the weighing funnel 11, 12, 13 is turned a little more than 90° by means of the turning notch 19, so that the existing first sample 7a in the weighing funnel is tipped out of the funnel for collection in a not-shown vessel. Thereafter, the above-mentioned oscillation or shaking movement of the weighing funnel is initiated in order to remove any pellets which may possibly be hanging in the weighing funnel 11, 12, 13.

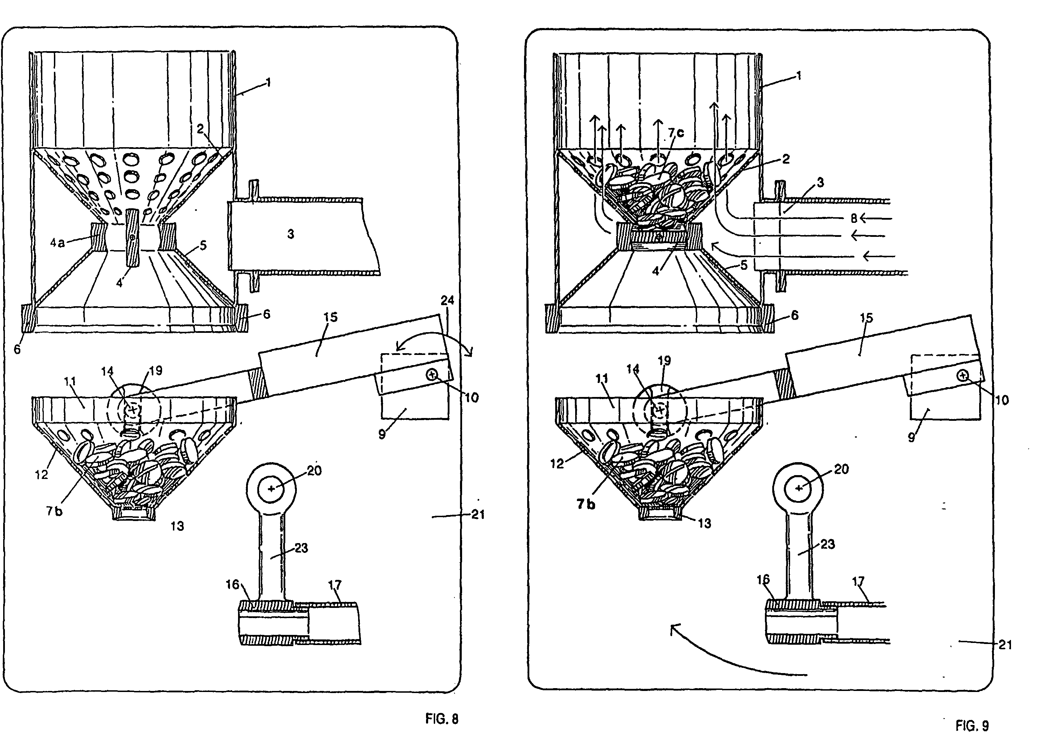

In a seventh step (see fig. 8), the weighing funnel is turned back to that position in which its axis is again substantially vertical, after which the rotary valve 4 in the ring 4a is opened to allow the second sample 7b to fall down into the weighing funnel 11, 12, 13 for the execution of a first weighing of the second sample 7b, as shown by the double arrow 24. The situation thus corresponds to that shown in figure 3, in that instead of the first sample 7a in fig. 3, it is now the second sample 7b which is about to be weighed.

The second sample 7b now follows a cycle which corresponds to that described above and as shown by steps 1 to 8 in figs. 3 to 8, and when the rotary valve 4 is turned back to the position shown in fig. 4, the tester according to the invention is ready to receive a third sample 7c. This new situation is shown in fig. 9, where the third sample 7c is deposited in the upper funnel 2.

It will be clear that by means of the described method and tester according to the invention, the possibility is presented for continuous testing of the current production of feed pellets without unnecessary delays, the reason being that the testing of the individual samples takes place while a subsequent sample is cooled and/or dried, said cooling/drying period being the most time-consuming during the testing process.

## Claims

1. Method for the testing of feed pellets which are extruded in a production plant, where samples (7a, 7b, 7c, --) of feed pellets are removed, cooled and dried before being tested for their consistency by going through the following steps:
a) A sample (7a, 7b, 7c, --) is weighed and the result is registered as a 1st weighing,
b) the sample (7a, 7b, 7c, --) is swirled around and blown through in order to remove dust and loosely-sitting particles from the sample,
c) the sample (7a, 7b, 7c, --) is weighed and the result is registered as a 2nd weighing,
d) 1st and 2nd weighings are compared as a measure of the consistency of the sample (7a, 7b, 7c, --), and
e) the sample (7a, 7b, 7c, --) is replaced by a subsequent sample (7b, 7c, 7d, --),
**characterized in that** the subsequent sample (7b, 7c, 7d, --) is cooled and dried concurrently with the above steps a) - e).

2. Tester for use in the execution of the method according to claim 1, said tester being of the kind having first pneumatic elements for cooling and drying and other pneumatic elements for blowing through and swirling a sample of feed pellets, weighing elements for weighing the relevant sample, storage elements for storing the results of the weighings and calculating elements for comparing the results of the weighings which are obtained before and after said blowing through and swirling of a sample, **characterized in that** the first pneumatic elements comprise a pipe piece (1) which in the position of use surrounds an upper container (2) with a perforated wall which, via a closable opening (4a),' is connected to a lower container (5) with a closed wall, and in the pipe piece (1) between the upper (2) and the lower container (5) a channel (3) opening into the pipe piece (1) for leading cooling/drying air (8) to and through the perforated wall of the upper container (2) and through a sample (7a, 7b, 7c, --) of feed pellets deposited in this container, and in that the weighing elements consist of a weighing funnel (11, 12, 13) with a perforated wall (12) which, at the bottom, has a sieve provided with a ring (13), said weighing funnel (11, 12, 13) being pivotably suspended at its upper edge around pins (14) in a forked weighing arm (15), the other end of which is connected via an axle (10) to a weighing cell (9) and can be turned between a first position, in which the weighing funnel abuts against and connects to the circumference of said opening of the lower container (5), and a second position in which the weighing funnel (11, 12, 13) is at a distance from said opening and can turn freely around the pins (14), in which second position the weighing cell (9) can be activated for the weighing of the sample (7a, 7b, 7c, --), and in that the other pneumatic elements comprise an air nozzle (16) with an air pipe (17) which are removable from the weighing funnel (11, 12, 13), said air nozzle and pipe in an upper position and in the first position of the weighing arm (15) lying up against the ring provided with a sieve (13), and being arranged to lead a stream of air (18, 27) through the sample (7a, 7b, 7c, --) deposited in the weighing funnel and in the lower container (5), and which in a lower position and in the second position of the weighing arm is at a distance from the ring (13) and permits the turning of the weighing funnel, and in that the tester has elements for turning the weighing funnel more than 90° away from a gravity-determined position in the weighing arm (15) for the pouring out of a sample (7a, 7b, 7c, --) deposited therein.

3. Tester according to claim 2, **character** **ized in that** the upper container is a downwardly-tapering funnel (2), and that the opening (4a) in the funnel (2) can be closed by means of a rotary valve (4) which can be turned around an axis which lies in the plane of the opening between a first position, in which the opening is blocked, and a second position in which the opening (4a) is open.

4. Tester according to claim 2, **characterized in that** the pins (14) are diametrically opposite each other and extend outwards from a first ring (11) along the upper edge of the weighing funnel, and in that the free end of the one pin (14) has a square portion which, in the second position of the weighing arm, engages in a turning notch (19) which is arranged to be able to turn the pin (14) and therewith also the weighing funnel (11, 12, 13) to an angle of more than 90°.

5. Tester according to claim 4, **characterized in that** the turning notch (19) in a turned position is arranged to be able to turn the pin (14) forwards and backwards in a small angular movement and thus impart oscillations or shaking movements to the weighing funnel (11, 12, 13).

6. Tester according to claim 2, **characterized in that** the air nozzle (16) is mounted on the end of an arm (23) which is suspended on an shaft (20), so that the air nozzle (16) with pipe (17) can be moved between the upper and the lower position.

7. Tester according to any of the foregoing claims, **characterized in that** the tester has take-off means from the calculating elements, said take-off means being configured to be able to supply the results of the consistency of the sample to and for the control of a production plant.

8. Tester according to any of the foregoing claims, c h a**racterized in that** the blowers for the pneumatic elements, the weighing cell (9), elements for movement of the weighing arm (15) and for the arm (23) for the air nozzle (16) and the pipe (17), the calculating elements and the means for the control of the tester's mutual sequence of functions, are disposed in a housing (22), and that the pipe piece (1) with the upper (2) and the lower container (5), the weighing arm (15) with weighing funnel (11, 12, 13) and the air nozzle (16) with the pipe (17) are disposed on the front (21) of the housing (22).

## Patentansprüche

1. Verfahren zum Prüfen von Futterpellets, die in einer Herstellungsanlage extrudiert werden, wobei Proben (7a, 7b, 7c, --) der Futterpellets entnommen, gekühlt und getrocknet werden, bevor sie auf ihre Konsistenz geprüft werden, indem sie die folgenden Schritte durchlaufen;
a) eine Probe (7a, 7b, 7c, --) wird gewägt, und das Ergebnis wird als erste Wägung registriert,
b) die Probe (7a, 7b, 7c, --) wird verwirbelt, und es wird durch sie hindurchgeblasen, um Staub und lose sitzende Teilchen von der Probe zu entfernen,
c) die Probe (7a, 7b, 7c, --) wird gewägt, und das Ergebnis wird als zweite Wägung registriert,
d) die erste und die zweite Wägung werden als Maß der Konsistenz der Probe (7a, 7b, 7c, -) verglichen, und
e) die Probe (7a, 7b, 7c, --) wird durch eine folgende Probe (7b, 7c, 7d, --) ersetzt,
**dadurch gekennzeichnet, dass** die folgende Probe (7b, 7c, 7d, --) zeitgleich zu den oben erwähnten Schritten a) - e) gekühlt und getrocknet wird.

2. Prüfeinrichtung zum Einsatz bei der Ausführung des Verfahrens nach Anspruch 1, wobei die Prüfeinrichtung von dem Typ ist, der erste pneumatische Elemente zum Kühlen und Trocknen sowie weitere pneumatische Elemente zum Hindurchblasen durch eine Probe von Futterpellets und zum Verwirbeln derselben, Wägeelemente zum Wägen der betreffenden Probe, Speicherelemente zum Speichem der Ergebnisse der Wägungen und Berechnungselemente zum Vergleichen der Ergebnisse der Wägungen aufweist, die vor und nach dem Hindurchblasen durch eine Probe und dem Verwirbeln derselben ermittelt werden, **dadurch gekennzeichnet, dass** die ersten pneumatischen Elemente ein Rohrteil (1) umfassen, das in der Einsatzposition einen oberen Behälter (2) mit einer Lochwand umgibt, der über eine verschließbare Öffnung (4a) mit einem unteren Behälter (5) mit einer geschlossenen Wand verbunden ist, und dadurch, dass sich in dem Rohrteil (1) zwischen dem oberen (2) und dem unteren Behälter (5) ein Kanal (3) in das Rohrteil (1) hinein öffnet, der Kühl/Trocken-Luft (8) zu der Lochwand des oberen Behälters (2) und durch sie hindurch sowie durch eine Probe (7a, 7b, 7c, -) von Futterpellets, die sich in diesem Behälter befindet, leitet, und dadurch, dass die Wägeelemente aus einem Wägetrichter (11, 12, 13) mit einer Lochwand (12) bestehen, der am Boden ein mit einem Ring (13) versehenes Sieb aufweist, wobei der Wägetrichter (11, 12, 13) an seinem oberen Rand um Zapfen (14) herum in einem gegabelten Wägearm (15) schwenkbar aufgehängt ist, dessen anderes Ende über eine Achse (10) mit einer Wägezelle (9) verbunden ist und zwischen einer ersten Position, in der der Wägetrichter am Umfang der Öffnung des unteren Behälters (5) anliegt und damit verbunden ist, und einer zweiten Position gedreht werden kann, in der der Wägetrichter (11, 12, 13) einen Abstand zu der Öffnung aufweist und sich ungehindert um die Zapfen (14) herum drehen kann, wobei in der zweiten Position die Wägezelle (9) zum Wägen der Probe (7a, 7b, 7c, --) aktiviert werden kann, und dadurch, dass die anderen pneumatischen Elemente eine Lüftdüse mit einem Luftrohr (17) umfassen, die von dem Wägetrichter (11, 12, 13) entfernt werden können, wobei die Luftdüse und das Rohr in einer oberen Position und der ersten Position des Wägearms (15) an dem mit einem Sieb (13) versehenen Ring anliegen und einen " Luftstrom (18, 27) durch die Probe (7a, 7b, 7c, --) leiten, die sich in dem Wägetrichter und in dem unteren Behälter (5) befindet, und die in einer unteren Position und der zweiten Position des Wägearms einen Abstand zu dem Ring (13) aufweist und das Drehen des Wägetrichters ermöglichen, und dadurch, dass die Prüfeinrichtung Elemente aufweist, mit denen der Wägetrichter um mehr als 90° aus einer durch die Schwerkraft bestimmten Position in dem Wägearm (15) gedreht wird, um eine Probe (7a, 7b, 7c, -) auszuschütten, die sich darin befindet.

3. Prüfeinrichtung nach Anspruch 2, **dadurch gekennzeichnet, dass** der obere Behälter ein sich nach unten verjüngender Trichter (2) ist, und dass die Öffnung (4a) in dem Trichter (2) mit einem Drehventil (4) verschlossen werden kann, das um eine Achse herum, die in der Ebene der Öffnung liegt, zwischen einer ersten Position, in der die Öffnung verschlossen ist, und einer zweiten Position, in der die Öffnung (4a) offen ist, gedreht werden kann.

4. Prüfeinrichtung nach Anspruch 2, **dadurch gekennzeichnet, dass** die Zapfen (14) einander diametral gegenüberliegen und sich von einem ersten Ring (11) am oberen Rand des Wägetrichters nach außen erstrecken, und dadurch, dass das freie Ende des einen Zapfens einen viereckigen Abschnitt aufweist, der in der zweiten Position des Wägearms in eine Dreheinkerbung (19) eingreift, die den Zapfen (14) und damit auch den Wägetrichter (11, 12, 13) in einen Winkel von mehr als 90° drehen kann.

5. Prüfeinrichtung nach Anspruch 4, **dadurch gekennzeichnet, dass** die Dreheinkerbung (19) den Zapfen (14) in einer gedrehten Position in einer geringfügigen Winkelbewegung vorwärts und rückwärts drehen kann und damit den Wägetrichter (11, 12, 13) in Schwingungen bzw. Schüttelbewegungen versetzen kann.

6. Prüfeinrichtung nach Anspruch 2, **dadurch gekennzeichnet, dass** die Luftdüse (16) an dem Ende eines Arms (23) angebracht ist, der an einer Welle (20) aufgehängt ist, so dass die Luftdüse (16) mit dem Rohr (17) zwischen der oberen und der unteren Position bewegt werden kann.

7. Prüfeinrichtung nach einem der vorangehenden Ansprüche, **dadurch gekennzeichnet, dass** die Prüfeinrichtung eine Abnahmeeinrichtung von den Berechnungselementen aufweist, wobei die Abnahmeeinrichtung so konfiguriert ist, dass sie die Ergebnisse der Konsistenz der Probe der und für die Steuerung einer Herstellungsanlage zuführt.

8. Prüfeinrichtung nach einem der vorangehenden Ansprüche, **dadurch gekennzeichnet**, dass die Gebläse für die pneumatischen Elemente, die Wägezelle (9), Elemente zum Bewegen des Wägearms (15) und für den Arm (23) für die Luftdüse (16) und das Rohr (17), die Berechnungselemente sowie die Einrichtung zum Steuern des Ablaufs von Funktionen der Prüfeinrichtung zueinander in einem Gehäuse (22) angeordnet sind, und dass das Rohrteil (1) mit dem oberen (2) und dem unteren Behälter (5), der Wägearm (15) mit dem Wägetrichter (11, 12, 13) sowie die Luftdüse (16) mit dem Rohr (17) an der Vorderseite (21) des Gehäuses angeordnet sind.

## Revendications

1. Procédé servant à tester des aliments granulés qui sont extrudés dans une installation de production, dans lequel des échantillons (7a, 7b, 7c, - -) d'aliments granulés sont enlevés, refroidis et séchés avant que leur homogénéité soit testée, ce procédé comprenant les étapes suivantes consistant à :
a) peser un échantillon (7a, 7b, 7c, --) et enregistrer le résultat en tant que premier pesage,
b) faire tourbillonner l'échantillon (7a, 7b, 7c, --) et souffler de l'air à travers celui-ci afin d'enlever de l'échantillon la poussière et les particules déposées à faible adhérence,
c) peser l'échantillon (7a, 7b, 7c, --) et enregistrer le résultat en tant que deuxième pesage,
d) comparer les premier et deuxième pesages sous la forme d'une mesure de l'homogénéité de l'échantillon (7a, 7b, 7c, --), et
e) remplacer l'échantillon (7a, 7b, 7c, --) par un échantillon suivant (7b, 7c, 7d, --),
**caractérisé en ce que** l'échantillon suivant (7b, 7c, 7d, --) est refroidi et séché simultanément aux étapes ci-dessus a) à e).

2. Testeur devant être utilisé lors de l'exécution du procédé selon la revendication 1, ledit testeur étant du genre comportant des premiers éléments pneumatiques de refroidissement et de séchage et d'autres éléments pneumatiques destinés à souffler de l'air à travers un échantillon d'aliments granulés et à le faire tourbillonner, des éléments de pesage destinés à peser l'échantillon approprié, des éléments de mémorisation destinés à mémoriser les résultats des pesages et des éléments de calcul destinés à comparer les résultats des pesages obtenus avant et après ladite opération de soufflage d'air à travers un échantillon et de tourbillonnement de celui-ci, **caractérisé en ce que** les premiers éléments pneumatiques comprennent une conduite (1) qui, dans la position d'utilisation, entoure un réceptacle supérieur (2) doté d'une paroi perforée qui, par l'intermédiaire d'une ouverture pouvant se fermer (4a), est raccordé à un réceptacle inférieur (5) doté d'une paroi fermée, et dans la conduite (1) entre le réceptacle supérieur (2) et le réceptacle inférieur (5), un passage (3) s'ouvrant dans la conduite (1) de façon à acheminer l'air de refroidissement/séchage (8) vers et à travers la paroi perforée du réceptacle supérieur (2) et à travers un échantillon (7a, 7b, 7c, --) d'aliments granulés déposés dans ce réceptacle, et en ce que les éléments de pesage sont constitués d'un entonnoir de pesage (11, 12, 13) doté d'une paroi perforée (12) qui comporte au fond un crible muni d'un anneau (13), ledit entonnoir de pesage (11, 12, 13) étant suspendu de manière pivotante au niveau de son bord supérieur autour d'axes (14) se trouvant dans un bras de pesage à fourche (15), dont l'autre extrémité est raccordée, par l'intermédiaire d'un axe (10), à une cellule de pesage (9), et pouvant être pivoté entre une première position, dans laquelle l'entonnoir de pesage est en butée contre la circonférence de ladite ouverture du réceptacle inférieur (5) et est relié à celle-ci, et une deuxième position dans laquelle l'entonnoir de pesage (11, 12, 13) est à une certaine distance de ladite ouverture et peut tourner librement autour des axes (14), dans laquelle deuxième position la cellule de pesage (9) peut être activée de façon à peser l'échantillon (7a, 7b, 7c, --), et en ce que les autres éléments pneumatiques comprennent une buse d'injection d'air (16) munie d'un tuyau d'arrivée d'air (17) qui peuvent être retirés de l'entonnoir de pesage (11, 12, 13), ladite buse d'injection d'air et ledit tuyau, dans une position supérieure et dans la première position du bras de pesage (15), se trouvant en haut en butée contre l'anneau muni d'un crible (13), et étant disposés de façon à acheminer un écoulement d'air (18, 27) à travers l'échantillon (7a, 7b, 7c, --) déposé dans l'entonnoir de pesage et dans le réceptacle inférieur (5), et qui, dans une position inférieure et dans la deuxième position du bras de pesage, sont à une certaine distance de l'anneau (13) et permettent le pivotement de l'entonnoir de pesage, et en ce que le testeur comporte des éléments destinés à pivoter en écartant l'entonnoir de pesage de plus de 90° par rapport à une position déterminée par la gravité dans le bras de pesage (15), afin de permettre le vidage de l'échantillon (7a, 7b, 7c, --) qui y est déposé.

3. Testeur selon la revendication 2, **caractérisé en ce que** le réceptacle supérieur est un entonnoir de section décroissante vers le bas (2), et en ce que l'ouverture (4a) de l'entonnoir (2) peut être fermée au moyen d'un robinet rotatif (4) pouvant tourner autour d'un axe se trouvant dans le plan de l'ouverture entre une première position, dans laquelle l'ouverture est bloquée, et une deuxième position, dans laquelle l'ouverture (4a) est ouverte.

4. Testeur selon la revendication 2, **caractérisé en ce que** les axes (14) sont diamétralement opposés l'un à l'autre et s'étendent vers l'extérieur depuis un premier anneau (11) situé le long du bord supérieur de l'entonnoir de pesage, et en ce que l'extrémité libre de l'axe (14) comporte une partie carrée qui, dans la deuxième position du bras de pesage, s'introduit dans une encoche de pivotement (19) disposée de façon à pouvoir pivoter l'axe (14) et avec lui également l'entonnoir de pesage (11, 12, 13) à un angle supérieur à 90°.

5. Testeur selon la revendication 4, **caractérisé en ce que** l'encoche de pivotement (19), dans une position pivotée, est disposée de façon à pouvoir pivoter l'axe (14) vers l'avant et vers l'arrière avec un petit mouvement angulaire et ainsi à impartir des oscillations ou des mouvements d'agitation à l'entonnoir de pesage (11, 12, 13).

6. Testeur selon la revendication 2, **caractérisé en ce que** la buse d'injection d'air (16) est montée sur l'extrémité d'un bras (23) qui est suspendu sur un arbre (20), de sorte que la buse d'injection d'air (16) munie du tuyau (17) peut être déplacée entre la position supérieure et la position inférieure.

7. Testeur selon l'une quelconque des revendications précédentes, **caractérisé en ce que** le testeur comporte un moyen d'extraction à partir des éléments de calcul, ledit moyen d'extraction étant configuré pour pouvoir délivrer les résultats d'homogénéité de l'échantillon à une installation de production en vue de la commander.

8. Testeur selon l'une quelconque des revendications précédentes, **caractérisé en ce que** les ventilateurs des éléments pneumatiques, la cellule de pesage (9) , les éléments de déplacement du bras de pesage (15) et du bras (23) de la buse d'injection d'air (16) et du tuyau (17), les éléments de calcul et le moyen de commande de la séquence de fonctions mutuelle du testeur, sont disposés dans un boîtier (22), et en ce que la conduite (1), avec les réceptacles supérieur (2) et inférieur (5) , le bras de pesage (15), avec l'entonnoir de pesage (11, 12, 13), et la buse d'injection d'air (16) avec le tuyau (17), sont disposés à l'avant (21) du boîtier (22).
